Europäisches Patentamt

European Patent Office

Office européen des brevets

(11) Publication number: **0 602 267 A1**

# EUROPEAN PATENT APPLICATION

(21) Application number: **92121352.6**

(22) Date of filing: **16.12.92**

(51) Int. Cl.5: **A61H 39/06**

(43) Date of publication of application:
**22.06.94 Bulletin 94/25**

(84) Designated Contracting States:
**DE FR GB**

(71) Applicant: **KABUSHIKI KAISYA ADVANCE**
**5-7, Nihonbashi Kobuna-cho**
**Chuo-ku Tokyo 103(JP)**

(72) Inventor: **Watanuki, Junichi**
**1-19-16-203, Toshima**
**Kita-ku, Tokyo(JP)**
Inventor: **Meguro, Yasuo**
**2-59-59, Minamidaira**
**Hino-shi, Tokyo(JP)**

(74) Representative: **Cohausz & Florack**
**Patentanwälte**
**Postfach 33 02 29**
**D-40435 Düsseldorf (DE)**

(54) Electric thermal treatment device.

(57) The present invention provides an electric thermal treatment device that does not cause a living body to feel an uncomfortable stimulus or suffer a burn. The electric thermal treatment device comprises a power supply (2), an electrothermal converter (4), a percutaneous heat conductor (6) that is joined with the electrothermal converter (4), has a heat conductivity ranging from 0.1 to 3.0 W $\cdot$ m$^{-1}$ $\cdot$ k$^{-1}$, and possesses viscosity, and a control unit (3) for controlling the temperature of the percutaneous heat conductor (6).

*Fig. 1*

EP 0 602 267 A1

BACKGROUND OF THE INVENTION

1. Field of the Invention

The present invention relates to an electric thermal treatment device.

2. Description of the Related Art

An electric thermal treatment device that gives a thermal stimulus to a lesion of a living body using an electrothermal conversion element has been proposed in recent years. This device substitutes for a thermotherapeutic procedure in which moxa is placed on a lesion of a living body, then burnt to give a thermal stimulus.

The electrothermal conversion element is a combination of a heating element that converts electric energy into heat and a heat conductor that supports the heating element and externally diffuses the heat from the heating element.

However, no specific consideration has been given to the heat conductivities of the heating element and heat conductor in the past. Therefore, the heat conductivities are, in general, considerably higher than the heat conductivity of a living body.

For instance, if the heat conductor is made of alumina ceramic, the heat conductivity is about 20 $W \cdot m^{-1} \cdot k^{-1}$. A living body consisting mainly of water has a heat conductivity of about 0.5 $W \cdot m^{-1} \cdot k^{-1}$. Thus, a difference between the heat conductivities becomes a two-digit value.

Therefore, when the heat conductor is brought into contact with or placed close to a living body, the temperature of the boundary surface of the living body increases due to a large difference between their heat conductivities. This causes the living body to feel an uncomfortable stimulus or suffer a burn.

An electric means, which can be adopted for a thermal stimulation procedure that gives a thermal stimulus similar to the effect of using a linear burning moxa or other natural moxa, has not been introduced. Furthermore, an alternative procedure or device, which is portable, allows a user to move his/her body during thermal stimulation, and ensures a long service life for a battery, has not been available. The introduction of an electric thermal treatment device having these advantages has been desired.

SUMMARY OF THE INVENTION

An object of the present invention is to overcome the aforesaid drawbacks of prior arts, and to provide an electric thermal treatment device that has no potential risk of causing an uncomfortable stimulus or a burn but gives a satisfactory warm feeling, and that serves as an alternative method or device which is portable, allows a user to move his/her body during hyperthermic stimulation, and ensures a long service life for a battery.

To achieve the above object, a construction described below is adopted. More particularly, an electric thermal treatment device according to the present invention, fundamentally, comprises a power supply; an electrothermal converting means; a percutaneous heat conducting means that is joined with the heat converting means, has a heat conductivity ranging from 0.1 to 3.0 $W \cdot m^{-1} \cdot k^{-1}$, and possesses viscosity; and a control means for controlling the temperature of the percutaneous heat conducting means.

In an electric thermal treatment device according to the present invention, an adhesive material that has a heat conductivity of, preferably, at least 0.1 $W \cdot m^{-1} \cdot k^{-1}$, more preferably, ranging from 0.1 to 3.0 $W \cdot m^{-1} \cdot k^{-1}$, or more preferably, ranging from 0.1 to 0.5 $W \cdot m^{-1} \cdot k^{-1}$, and possesses flexibility is employed as a percutaneous heat conducting means placed between a heating means or a heat conducting means and a living body. Owing to this construction, concentration of heat on a surface of a living body on a boundary with an electrothermal converting means can be avoided. Therefore, the heat penetrates the living body smoothly and mildly. An electric thermal treatment device thus realized involves no risk of causing an uncomfortable stimulus or a burn, which is a drawback of the prior arts, and provides a satisfactory warm feeling.

BRIEF DESCRIPTION OF THE DRAWINGS

Figure 1 is a cross-sectional diagram showing a construction of an embodiment of an electric thermal treatment device according to the present invention;
Figure 2 shows a construction of another embodiment of an electric thermal treatment device of the present invention together with a cross section of part of the embodiment;

Figure 3 shows a construction of another embodiment of an electric thermal treatment device of the present invention together with a cross section of part of the embodiment;

Figure 4 is an oblique view of an embodiment of an electric thermal treatment device of the present invention with a percutaneous heat conducting means removed;

Figure 5 is an explanatory diagram of an embodiment of a corded electric thermal treatment device according to the present invention;

Figures 6 and 7 show an electrothermal converting means employed for an electric thermal treatment device according to the present invention;

Figure 8 is a block diagram showing an example of a construction of a control means employed for an electric thermal treatment device according to the present invention;

Figure 9 is an explanatory diagram showing a state in which an electric thermal treatment device according to the present invention is put into practical use;

Figure 10 is a graph showing a thermal characteristic in a conventional linear burning moxa treatment, i.e., Sen-nen-kyu (conventional moxibustion system);

Figure 11 is a graph showing an example of a thermal characteristic of an electric thermal treatment device according to the present invention; and

Figure 12 shows the relation between the input pulse and the heating temperature in an electric thermal treatment device according to the present invention.

## DETAILED DESCRIPTION OF THE PREFERRED EMBODIMENTS

Embodiments of the present invention will be described in detail with reference to the drawings.

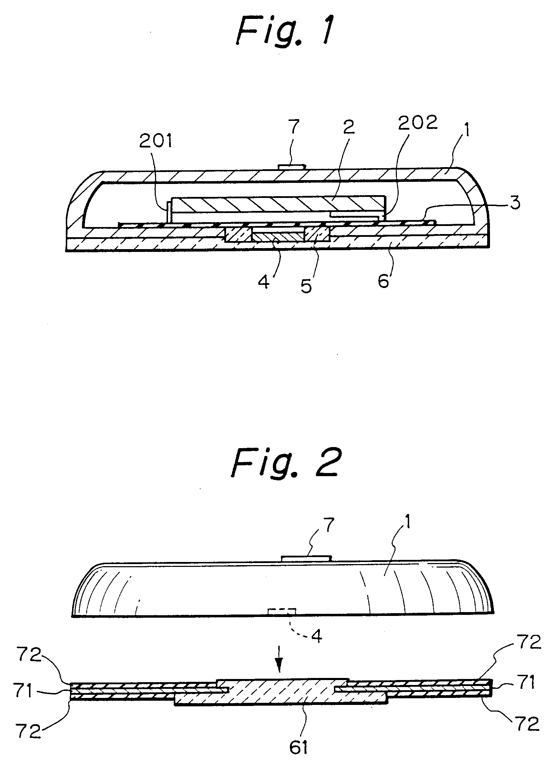

Figure 1 is a cross-sectional diagram showing an example of a construction of an electric thermal treatment device according to the present invention. The electric thermal treatment device comprises a power supply 2, an electrothermal converting means 4, a percutaneous heat conducting means 6 that is joined with the electrothermal converting means 4, has a heat conductivity ranging from 0.1 to 3.0 $W \cdot m^{-1} \cdot k^{-1}$, and possesses viscosity, and a control means 3 for controlling the temperature of the percutaneous heat conducting means 6. In the present invention, the heat conductivity k is represented as a quantity of heat flowing for one second over 1 $m^2$ of a plate that is 1 mm thick and has a temperature difference of 1°C between the sides. The unit is $W \cdot m^{-1} \cdot k^{-1}$.

As seen from Figure 1, an embodiment of an electric thermal treatment device according to the present invention is a portable electric thermal treatment device using a battery or other power supply. The present invention will be further detailed in conjunction with Figure 1.

Reference 1 denotes a main body case that is made of a plastic or resin.

Reference 2 denotes a battery that is a button or coin type primary or secondary small battery such as a nickel-cadmium battery.

Reference 3 denotes an electronic circuit board on which circuits are mounted to control supply of electric energy to a heating element.

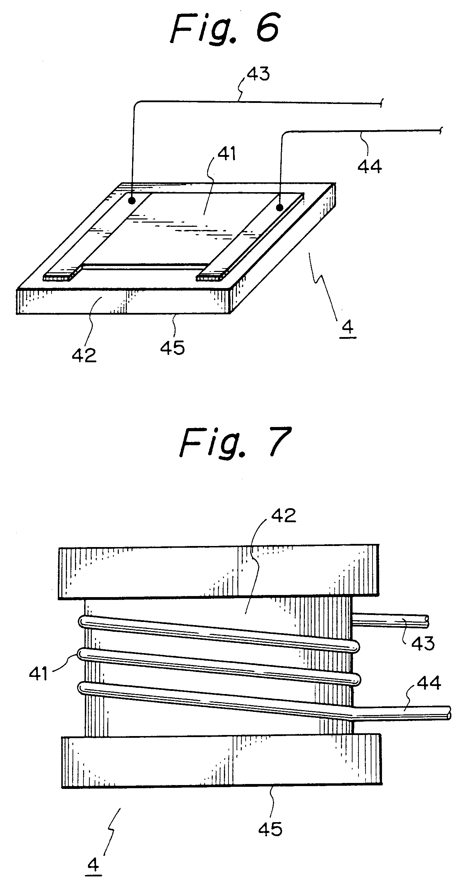

Reference 4 denotes an electrothermal converting element that is realized by a heating element made by printing or baking an aluminous or ceramic heat conductor with silver and palladium or by winding a ceramic heat conductor with an heating element such as a platinum wire or a Nichrome wire. Figures 6 and 7 show examples of specific shapes of the electrothermal converting element. Figures 6 and 7 will be described later.

Reference 5 denotes a holder for holding and fixing the electrothermal converting element 4 onto the bottom of the main body case 1. Preferably, the holder is made of a heat preserving and heat insulating material.

Reference 6 denotes a percutaneous heat conductor made of a base material of gel. The gel base has the aforesaid composition, and possesses viscosity, flexibility, and a water holding property.

Reference 7 denotes an operation switch. An operator uses this switch 7 to turn on or off current supply to the heating element or adjust an amount of supply current.

The operation switch 7 is formed on the top of the main body case 1. The small battery 2 and electronic circuit board 3 are placed in the main body case 1. The small battery 2 and electronic circuit board 3 are connected via conductive terminals 201 and 202.

The electrothermal converting element 4 held with the holder 5 is placed under the bottom of the electronic circuit board 3. Part of the holder 5 and electrothermal converting element 4 forms part of the bottom of the main body case 1. The holder 5 and electrothermal converting element 4 may project slightly from the bottom of the main body case 1.

The percutaneous heat conductor 6 is adhered all over the bottom of the main body case 1. The percutaneous heat conductor 6 may be adhered just before use and may be disposable. In the present invention, the heat conductivity of the percutaneous heat conductor 6 ranges, preferably, from 0.1 to 0.5 $W \cdot m^{-1} \cdot k^{-1}$. It is preferred that the surface or inside of the percutaneous heat conductor 6 be made of an adhesive material having flexibility.

The percutaneous heat conductor 6 employed for the present invention is made of a base material that is a moisture holding material or a hydrophilic high polymer gel compound. In particular, a hydrophilic high polymer gel compound consisting mainly of a polyalcohol is preferred.

A feature of the present invention is that a gel base is placed as a percutaneous heat conductor between a heating element or a heat conductor, and a living body. Preferably, the gel base contains a large amount of non-volatile liquid such as ethylene glycol, propylene glycol, and glycerin (heat conductivity of $0.285 \ W \cdot m^{-1} \cdot k^{-1}$). Therefore, the heat conductivity of the overall gel base becomes similar to that of a living body. Consequently, heat transmitted through the gel base does not concentrate on a boundary surface of a living body but penetrates the living body smoothly. A gel sheet has a low thermal diffusivity, which, therefore, preserves heat efficiently. Eventually, remaining heat can be utilized fully. This is ideal for thermotherapy. Furthermore, the gel is neither evaporated nor dried, and, therefore, can be used for a prolonged period of time. The thickness and area of a gel base vary depending on a temperature used for thermotherapy and an area to be treated, and are not limited to any specific values. An appropriate thickness ranges, for example, from 0.2 to 5 mm.

The gel base is flexible and adhesive to the skin. Therefore, an effective contact area can be maintained sufficiently. When an entire device is to be adhered to the skin, the property of the gel that adheres to the skin will be helpful.

Next, embodiments of a base that is made of a hydrophilic high polymer gel compound in the present invention will be described.

Preferably, some natural resin polysaccharides including karaya gum, tragacanth gum, and zansan gum, partly saponified polyvinyl alcohol, polyvinyl formal, polyvinyl methyl ether and its copolymers, polyvinyl pyrolidone, vinyl resins including polyvinyl methacrylate, polyacrylic acid and its sodium salts, polyacrylamide and its partly hydrolyzed substrates, partly saponified polyacrylic ester, poly (acrylic acid - acrylamide)-acrylic resins, and other hydrophilic natural and synthetic resins are softened and plasticized with a polyalcohol including propylene glycol, ethylene glycol, and glycerin, and are then made into a flexible gel film or sheet having an autoshaping property and a property of adhering to the skin.

Polymers such as polyol and polyisocyanate which have flexibility and viscosity by themselves (refer to known examples mentioned later) are also preferred because their thermal characteristics are within the aforesaid range.

Needless to say, a thermal characteristic can be finely controlled by adding an electrolyte.

The property of adhering to the skin may be intensified with addition of an adhesive.

Next, preferred examples of the base material for the present invention will be listed.

Example 1

| Component | Multi-purpose content | Range of percentage content |
|---|---|---|
| Polyacrylamide | 30 (%) | 10 to 40(%) |
| Glycerin | 68 | 60 to 90 |
| N, N'-diallyltartaramide | 1 | 0.3 to 5 |
| or | | |
| N, N'-methylenebisacrylamide | 1 | 0.3 to 5 |
| Sodium chloride | 1 | 0.5 to 10 |
| or | | |
| Potassium citrate | 4 | 0.3 to 10 |

Preparation: the above components are mixed, then stirred under given conditions. Then, the mixture is heated at a given temperature for several hours to several tens of hours, and thus aged. The gel base thus produced has a heat conductivity of 0.3 $W \cdot m^{-1} \cdot k^{-1}$.

Example 2

| Acrylic acid | 10 | 5 to 15 |
|---|---|---|
| Glycerin | 84 | 80 to 95 |
| N, N'-diallyltartaramide | 1 | 1 to 2 |
| or | | |
| N, N'-methylenebisacrylamide | 1 | 1 to 2 |
| Potassium citrate | 4 | 1 to 5 |
| Ammonium persulfate | < 0.1 | < 0.1 |
| or | | |
| Sodium persulfate | < 0.1 | < 0.1 |
| Sodium pyrosulfite | < 0.1 | < 0.1 |
| or | | |
| Ferrous sulfate | < 0.1 | < 0.1 |

Preparation: the above components are mixed and stirred, then heated under the same conditions as Example 1. The produced gel base has a heat conductivity of 0.3 $W \cdot m^{-1} \cdot k^{-1}$.

Example 3

11 parts by weight of butyl acrylate, and 11 parts of methoxy-polyethylene glycol methacrylate (the number of reduplication of ethylene oxide, n, is 23) are resolved into 34 parts of benzene, then replaced with nitrogen within the system. Then, 0.03 parts of azobisisobutyronitrile is added as an initiator to the mixture, then copolymerized with nitrogen at 80°C for five hours.

Example 4

| Component | Content (weight percentage) |
|---|---|
| (Isobutylene/maleic anhydride) Alternate copolymer | 24 |
| Dipropyrene glycol or propylene glycol | 37 |
| Concentrated glycerin | 9 |
| Polyglycerinpolyglycidyleher | 4 |
| Sodium chloride (pH regulator) | 5 |
| Purified water | 20 |
| Preparation: the above components are mixed, then heated under the same conditions as those in Example 1. The thus produced gel base has a heat conductivity of 0.3 $W \cdot m^{-1} \cdot k^{-1}$. | |

Other gel bases

Some preferred embodiments of the gel sheet or film for the present invention have been described above. Diverse hydrophilic high polymer materials are known as materials for electrodes to be placed on a living body, any of which can be used selectively. For instance, any of hydrophilic high polymer materials disclosed in the publications listed below can be made into a thermally-characterized gel base by adjusting its content of polyalcohol.

Japanese Examined Patent Publication No. 2-9074
Japanese Unexamined Patent Publication No. 52-95895
Japanese Unexamined Patent Publication No. 54-77489
Japanese Unexamined Patent Publication No. 55-52742
Japanese Unexamined Patent Publication No. 55-81635
Japanese Unexamined Patent Publication No. 56-15728
Japanese Unexamined Patent Publication No. 56-36939
Japanese Unexamined Patent Publication No. 56-36940
Japanese Unexamined Patent Publication No. 56-60534
Japanese Unexamined Patent Publication No. 56-89270
Japanese Unexamined Patent Publication No. 56-143141
Japanese Unexamined Patent Publication No. 57-28505
Japanese Unexamined Patent Publication No. 57-49431
Japanese Unexamined Patent Publication No. 57-52463
Japanese Unexamined Patent Publication No. 57-55132
Japanese Unexamined Patent Publication No. 57-131428
Japanese Unexamined Patent Publication No. 57-1604064
Japanese Unexamined Patent Publication No. 57-166142
Japanese Unexamined Patent Publication No. 57-168675
Japanese Unexamined Patent Publication No. 57-4569
Japanese Unexamined Patent Publication No. 58-10066
Japanese Unexamined Patent Publication No. 62-86076
Japanese Unexamined Patent Publication No. 62-139628
Japanese Unexamined Patent Publication No. 62-270134
Japanese Unexamined Patent Publication No. 2-80030
Japanese Unexamined Patent Publication No. 54-80689
Japanese Unexamined Patent Publication No. 56-135706
Japanese Unexamined Patent Publication No. 56-138603
Japanese Unexamined Patent Publication No. 57-93305
Japanese Unexamined Patent Publication No. 57-179413

Japanese Unexamined Patent Publication No. 57-185309

As described above, the gel base of the present invention is made of a hydrophilic high polymer that has given thermal properties and possesses flexibility and viscosity. The gel base is preferably softened and plasticized with a polyalcohol. The materials made into the gel base are not limited to any specific ones. The composition of the gel base is determined in consideration of compatibility with the skin, and, if necessary, of heat conductivity and heat capacity. The gel base may be disposable or replaced with a new one at every use.

The shape and construction of a heating element or a heat conductor are not limited to specific ones. The heating element or heat conductor may be constructed to have a film-like or disk-like shape, or any other shape as long as it is joined with an electrothermal converting means to conduct heat.

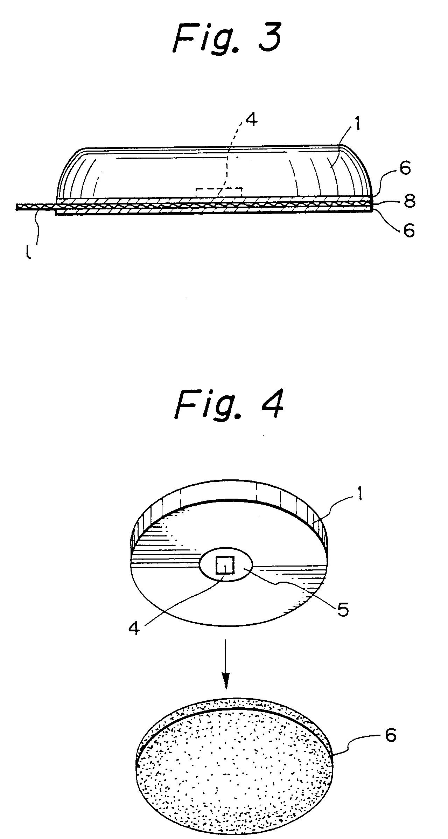

A percutaneous heat conducting means 6 made of the aforesaid raw materials according to the present invention is shaped like a disk having a diameter of, for example, 30 to 50 mm as shown in Figure 4. Then, the percutaneous heat conducting means 6 is brought partly into contact with and fixed to an electrothermal converting means 4 that is held by a holder 5 and appears from an opening formed on the bottom of a main body case 1 of an electric thermal treatment device according to the present invention.

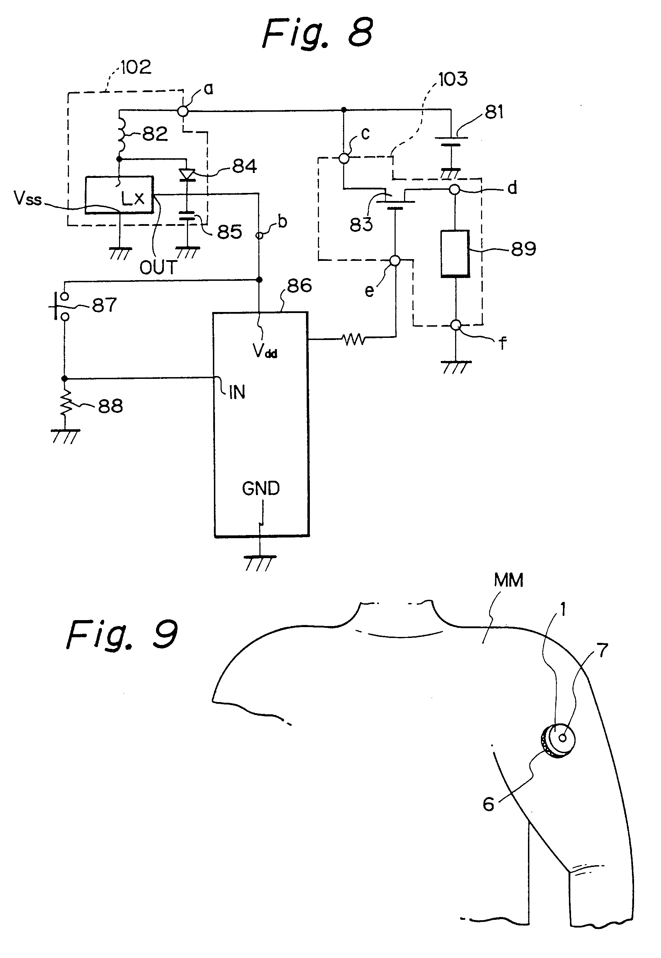

Next, the other side of the percutaneous heat conducting means 6 is adhered to a lesion of a living body. The main body case 1 is held and adhered to the lesion of the living body in one united body owing to the percutaneous heat conducting means 6. The state is shown in Figure 9. In the figure, the main body case 1 is adhered to the left anterior arm of a human body (MM).

Next, a control button 7 formed on the top of the main body case 1 is operated to power on. Current from a small battery is supplied to the electrothermal converting means 4 via a control means on an electronic circuit board 3. Then, the elecrothermal converting means 4 releases heat for a given period of time. Heat released from the electrothermal converting means 4 is fed to the lesion of the living body via the percutaneous heat conducting means 6. The percutaneous heat conducting means 6 preserves heat and gives a comfortable warm feeling to a wide area of the living body.

Figure 2 shows the second embodiment of the present invention.

A main body case 1 and its internal construction are identical to those of an embodiment shown in Figure 1. Reference 61 denotes a percutaneous heat conducting means composed of a gel base having low viscosity. The percutaneous heat conducting means 61 is arranged only around an electrothermal converting means 4. The outer margin of the percutaneous heat conducting means 61 is surrounded with adhesive layers 63 each having high viscosity. The adhesive layers 63 are composed of adhesive 72 applied on both sides of a ring type supporting member 71. The composition of the adhesive 72 is identical to or similar to that of a known adhesive tape.

In the embodiment shown in Figure 2, the main body case 1 is adhered to a lesion of a living body with the adhesive 72. This embodiment is preferred from the viewpoint of improved adhesion. Although the gel base of the percutaneous heat conducting means 61 has low viscosity, the adhesion of the adhesive 72 brings the percutaneous heat conducting means 61 into contact with the living body satisfactorily.

Figure 3 shows the third embodiment of the present invention.

A housing 1 and its internal construction are identical to those of an embodiment shown in Figure 1. A percutaneous heat conducting means 6 is composed of a two-layered gel base which is intervened with nonwoven cloth, tulle net, or other cloth 8. When the thickness of the percutaneous heat conducting means 6 is as thin as 5 mm or less, the percutaneous heat conducting means 6 may tear. The use of the cloth 8 reinforces the percutaneous heat conducting means 6, and no tearing occurs.

Furthermore, if the cloth 8 alone comes out of the percutaneous heat conducting means 6 slightly or by a length 1 in Figure 3, the percutaneous heat conducting means 6 can be peeled off from the main body 1 effortlessly. This improves ease of use.

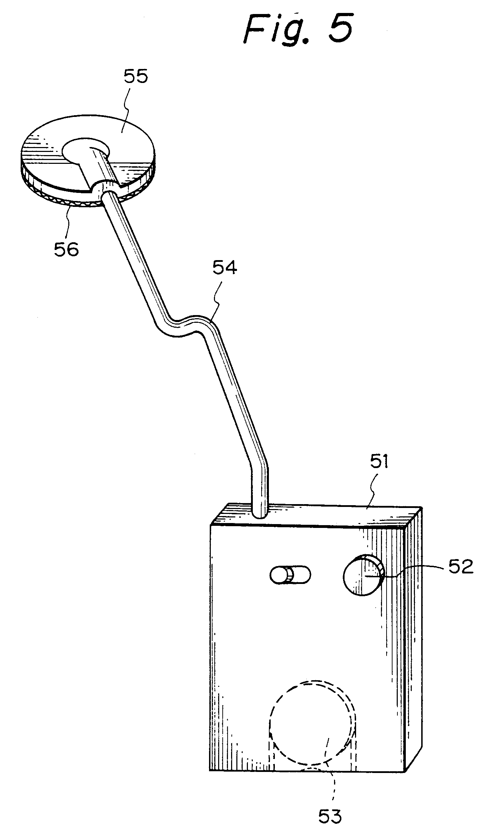

The aforesaid embodiments are examples of a cordless electric thermal treatment device. In Figure 5, however, a control unit 51 comprising a small battery and a control circuit, a heating element, a heating element case 55 or a main body case for encapsulating the heating element, and a percutaneous heat conducting means 56 are connected with an electric lead wire 54. In the control unit 51, a battery 53 is placed on the bottom, and an adjustment knob 52 is formed on the front surface. The electric lead wire 54 is sheathed with vinyl for electrical insulation. On the bottom of the heating element case 55, the heating element and heat conductor are attached to face outward. The bottom of the heating element case 55 is identical to that of the main body case 1 shown in Figure 4. The battery 53 may be of D, C, AA, AAA, or a smaller type, or may be replaced by a mains AC power supply. 56 denotes a percutaneous heat conducting means which is composed of a gel base. The shape and construction of the percutaneous heat conducting means 56 are identical to those of the aforesaid embodiments.

Figures 1 to 3 show so-called cordless devices in each of which a small battery control circuit, a heating element, and a percutaneous heat conductor are united as one body and adhered to a living body for treatment. In the embodiment shown in Figure 5, the heating temperature of the heating element is controllable remotely.

In another embodiment, a gel base having no viscosity is used for a percutaneous heat conductor. The entire main body is adhered to the skin with adhesive tape.

Next, an electrothermal converting means 4 employed in the present invention will be described. The electrothermal converting means includes an electrothermal converting element of an appropriate type. Embodiments of the electrothermal converting means will described in conjunction with Figures 6 and 7. Reference 41 denotes a heating element made of silver and palladium. Reference 42 denotes a heat conductor made mainly of alumina or other ceramic. References 43 and 44 denote electric lead wires extending to a control means and a battery. The heating element 41 is printed or baked on the heat conductor 42. The electric lead wires 43 and 44, and the heat conductor 41 are joined by welding. Figure 7 shows a bobbin type heat conductor 42 wound with a heat conductor that is a platinum wire or a tungsten wire. References 43 and 44 are electric lead wires which are, similarly to the above embodiment, connected to a control means and a battery.

Reference 45 in Figure 6 or 7 denotes a portion that appears on the bottom when an electrothermal converting element is incorporated in a heating element case 55 shown in Figure 5.

The portion that appears on the bottom of the heating element case is not limited to the above surface of the heat conductor 42 but may be the surface of the heating element.

Next, a control means according to the present invention will be described.

An electric thermal treatment device according to the present invention is divided into a type in which a heating element and a control unit are connected with a cord, and a cordless portable type. The electric thermal treatment device itself is controlled according to the same method. The construction and operation of a control means 100 for an electric thermal treatment device of the cordless portable type will now be described.

In the present invention, an electric thermal treatment device 1 is controlled so that a percutaneous heat conducting means 6 will provide substantially the same heating temperature characteristics as Sen-nen-kyu (conventional moxibustion) and so-called a linear burning moxa treatment device in which natural moxa is employed. Furthermore, using a small battery, current must be controlled effectively to continue treatment for a prolonged period of time.

To achieve this object, as shown in Figure 8, the control means for the electric thermal treatment device 1 of the present invention includes a microcomputer 86 that adjusts and controls a small current fed from a small battery 81 to make the most of the small current for the purpose of the present invention.

Furthermore, the control means 100 for the electric thermal treatment device 1 of the present invention is provided with a boosting means 102 that boosts the output voltage of the small battery 81 and produces the voltage required for driving the microcomputer 86. In addition, a heating control circuit 103 for allowing the electrothermal converting means 4 to release heat is incorporated.

A more specific construction of the control means 100 according to the present invention will now be described in detail.

A plus electrode of a small battery 81 is connected to one end of a coil 82 forming part of the boosting means 102 via a terminal a and to a drain of a field-effect transistor (FET) 83 forming a heating control means 103, via a terminal c.

On the other hand, the source of the FET 83 is connected to one end of a heating element 89 via a terminal d.

The other end of the coil 82 is connected to a switching terminal Lx of a DC/DC converter chip 90 and to an anode of a diode 84.

A cathode of the diode 84 is connected to a voltage output terminal OUT of the DC/DC converter chip 90, and to a power input terminal Vdd and one end of a switch 87 of the microcomputer 86, via a terminal b.

An output terminal of the microcomputer 86 is connected to a gate of the FET 83 of the heating control means 103, via a terminal e.

The manual switch 87 for manipulating the control means 100 is connected at one end to the power input terminal Vdd. The other end of the manual switch 87 is connected to one end of a resistor 88 and to an input terminal IN of the microcomputer.

The other end of a capacitor 85 and the other end of the resistor 88 are connected to the minus electrode of the battery 81.

The other end of the heating element 89 is connected to the minus electrode of the battery 81 via a terminal f.

The ground Vss of the DC/DC converter 90 and the ground GND of the microcomputer are connected to the minus electrode of the battery 81.

The battery 81 may be a button type Ni-Cd battery (1.2V. Rated capacitor: 50 mAh). The DC/DC converter 90 incorporates an oscillator, a chopper, a voltage comparator, and a reference voltage source. The reference voltage source and voltage comparator correct a variation in an output to provide a constant voltage. In practice, a DC/DC converter includes a coil, a diode, and a capacitor, which are installed externally. Despite the above construction, the DC/DC converter 90 is called a converter. The DC/DC converter may, for example, be an RH5RC series product (manufactured by Ricoh Co., Ltd.)

More specifically, in the control unit 100 according to the present invention, the microcomputer 86 is incorporated. Assuming that the operating voltage of the microcomputer 86 is 5V, if an output voltage of the small battery 81 is usually 1.2V, the microcomputer 86 does not operate. In the present invention, therefore, the boosting means 102 having the aforesaid construction is employed so that the microcomputer 86 will be actuated with the small current fed from the small battery 81.

With program control of the microcomputer 86, pulsating current is extracted from the battery 81. Then, the coil 82 generates intermittent and instantaneous high voltage. Then, the capacitor 85 stores the high voltage. Thus, a given boosted voltage necessary for driving the microcomputer 86 is produced.

On the other hand, a heating control circuit 103 of the present invention comprises a heating resistor 89 installed in an electrothermal converting means 4 and a current control circuit. The current control circuit is, for example, realized with a MOSFET 83.

Furthermore, it is preferred that the current control circuit including the MOSFET 83 and the electrothermal converting means 4, which make up the heating control circuit 103, are not intervened with any electric or electronic part but are connected directly with each other using a wire.

The aforesaid construction is preferably for effective release of heat using a very small pulsating current when a small battery is used as a power supply.

To be more specific, an on-state resistance of a current control circuit or a MOSFET 83 in the present invention is, preferably, smaller than an on-state resistance of a heating resistor 89.

The gate terminal of the MOSFET 83 forming the current control circuit is connected to the output terminal of the microcomputer 86. According to a given program stored in the microcomputer 86, a given pulse signal is fed to the gate terminal. Thereby, the current control circuit is turned on. While the pulse signal is being fed to the gate terminal, a given current is supplied from the small battery 81 to the heating resistor 89.

In the present invention, the current control circuit is controlled with a pulse that is supplied by the microcomputer 86 and has a given duration.

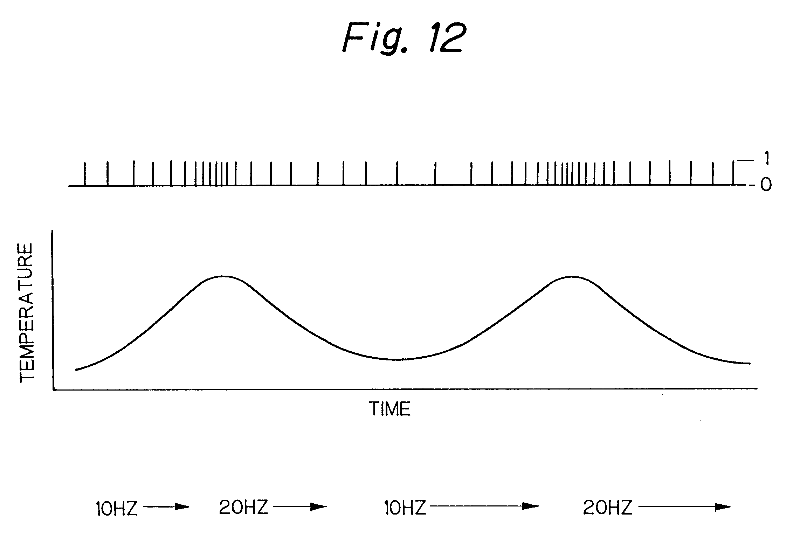

The operation of the microcomputer 86 of the present invention will now be described. A heating value or temperature of the heating resistor 89 is proportional to a current which the MOSFET 83 forming the current control circuit supplies to the heating resistor 89. In short, the heating value or temperature is proportional to a time interval in which the MOSFET 83 is on. In the present invention, a pulse generation cycle may be varied. As shown in Figure 12, the heating value or temperature may be set to a higher value for dense pulse durations and to a lower value for sparse pulse durations.

In the present invention, the heating temperature of the heating resistor 89 can be set to any value by varying an on-stage duty ratio or pulse duration for the MOSFET 83. Furthermore, an intended pattern of temperature variation can be produced.

In the present invention, a duty ratio or a pulse duration of each control pulse fed to the MOSFET 83 forming the current control circuit varies as time passes. Preferably, the duty ratio or pulse duration of the pulse varies as time passes so that the on-state time interval or cycle will extend.

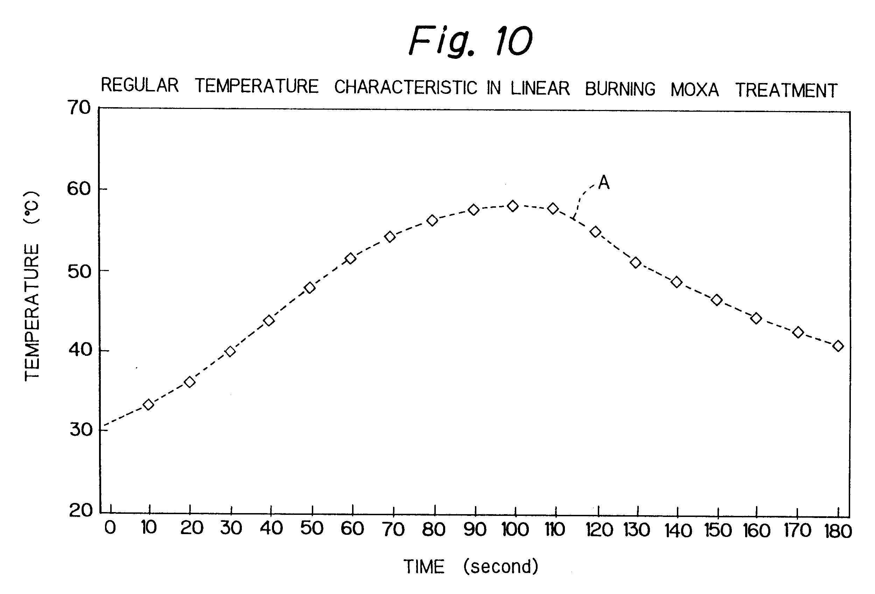

Figure 10 shows a curve indicating a temperature rise in a heating element that is designed for a known linear burning moxa treatment which uses natural moxa as a heating material placed in contact with the skin.

A graph A of Figure 10 indicates a temporal function of a temperature change characteristic of the heating element. Past experiments demonstrate that this temperature change characteristic is suitable for a human body.

Then, the present invention attempts to provide the same temperature change characteristic as that provided by the linear burning moxa treatment. Since the pulse duration of a control pulse to be fed to the MOSFET 83 is modified as shown in Figure 12, a temperature change characteristic similar to an ideal temperature change characteristic for the linear burning moxa treatment can be attained.

In the present invention, a program for generating a control pulse shown in Figure 12 is implemented in the microcomputer 86. Incidentally, in Figure 12, the duty ratio is held constant, and the frequency is varied cyclically in the range from 10 to 20 Hz.

The above temperature change characteristic is introduced merely as an example. The electric thermal treatment device of the present invention can adopt a heating method in which various temperature change characteristics can be provided by changing programs existent in the microcomputer 86.

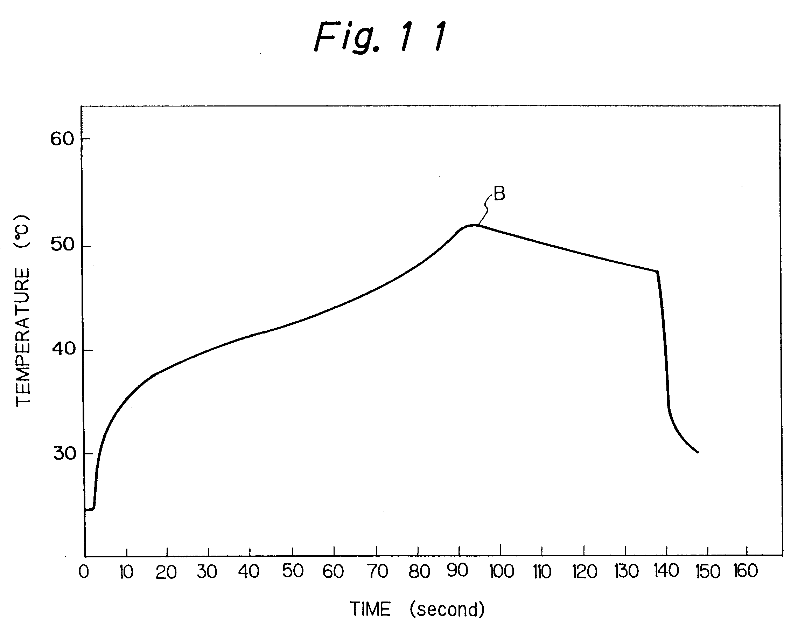

In the electric thermal treatment device of the present invention, the microcomputer 86, preferably, includes a repetitive operation facility for repeating a temperature change so that, as shown in Figure 11, a given low temperature is raised to a given high temperature in a given period of time, then, the high temperature is dropped to a given low temperature.

In a known linear burning moxa treatment device, a temperature change such as that shown in Figure 11 is generally repeated at least three times. Therefore, in the present invention, a program is, preferably, written to repeat a temperature change plotted as a graph B of Figure 11, three times.

The operation of the aforesaid circuit will now be described.

When voltage of a battery 81 is fed to a DC/DC converter 90 via a coil 82, the DC/DC converter 90 is actuated. When current flowing through a coil 82 is interrupted, a back electromotive force occurs. The back electromotive force is stored in a capacitor 85 via a diode 84. The voltage stored in the capacitor 85 enters a power input terminal Vdd of a microcomputer 86 via a terminal b. Thus, the voltage stored in the capacitor 85 is fed to the microcomputer 86. In this state, when a switch 87 is pressed, voltage is fed to an input terminal IN of the microcomputer 4. Then, the microcomputer 86 starts running. The microcomputer 86 outputs a drive pulse to a gate of a FET 83 via a terminal e according to a program stored in a storage means.

The FET 83 switches between terminals c and d according to whether a drive pulse is supplied. The frequency of a drive pulse ranges, for example, from 1 to 50 Hz. The frequency or duty ratio of a drive pulse is not restricted to any specific value.

When the terminals c and d are connected, energy of the battery 81 is fed to a heating element 89 via the FET 83. When the terminals c and d are cut off, supply of battery energy to the heating element 89 is blocked.

The heating element releases heat at a temperature that is proportional to the frequency or duty ratio of a drive pulse. Thus, the heating temperature is controlled according to the frequency or duty ratio of a drive pulse.

Since the battery 81 and the heating element 89 are disconnected from each other frequently, a voltage drop across the battery 81 is extreme. The DC/DC converter 90 compensates for the voltage drop so that a constant voltage will be supplied to the power supply Vdd of the microcomputer all of the time. This allows the heating element to release heat for a time interval required for thermotherapy.

As described so far, according to the present invention, a gel base is employed for a percutaneous heat conductor. Therefore, a warm feeling can be given continuously over a wide area without causing an uncomfortable thermal stimulus similar to a sting caused by a needle on the skin, or a burn.

## Claims

1. An electric thermal treatment device for thermotherapy, comprising:
   a power supply;
   an electrothermal converting means;
   a percutaneous heat conducting means that is joined with said electrothermal converting means, comprises an adhesive material having a heat conductivity ranging from 0.1 to 3.0 $W \cdot m^{-1} \cdot k^{-1}$; and control means for controlling the temperature of said electrothermal convering means.

2. An electric thermal treatment device according to claim 1, wherein said heat conductivity of said percutaneous heat conducting means ranges from 0.1 to 0.5 $W \cdot m^{-1} \cdot k^{-1}$.

3. An electric thermal treatment device according to claim 1 or 2, wherein said percutaneous heat conducting means includes an adhesive material that has flexibility.

4. An electric thermal treatment device according to claim 1, wherein said percutaneous heat conducting means is composed of a base material that is a hydrophilic high polymer gel compound.

5. An electric thermal treatment device according to any of claims 1 to 3, wherein said electric thermal treatment device has a structure that adheres to the skin as a whole.

6. An electric thermal treatment device according to claim 1, wherein said power supply is a portable battery.

7. An electric thermal treatment device according to claim 1, wherein said control means includes a microcomputer.

8. An electric thermal treatment device according to claim 7, wherein said control means includes a boosting means that boosts an output voltage of said battery to produce a voltage necessary for driving said microcomputer.

9. An electric thermal treatment device according to claim 7, wherein said control means includes a heating control circuit that allows said electrothermal converting means to release heat.

10. An electric thermal treatment device according to claim 9, wherein said heating control circuit comprises a heating resistor included in said electrothermal converting means, and a current control circuit.

11. An electric thermal treatment device according to claim 10, wherein said current control circuit is realized with a MOSFET.

12. An electric thermal treatment device according to claim 10, wherein said current control circuit and said heating resistor included in said electrothermal converting means, which make up said electrothermal control circuit, are connected directly to each other using a wire.

13. An electric thermal treatment device according to any of claims 10 to 12, wherein an on-state resistance of said current control circuit is set to a smaller value than an on-state resistance of said heating resistor.

14. An electric thermal treatment device according to claim 10, wherein said current control circuit is controlled with a pulse having a given duration.

15. An electric thermal treatment device according to claim 14, wherein a duty ratio of said pulse varies as time passes.

16. An electric thermal treatment device according to claim 15, wherein said duty ratio of said pulse varies as time passes so that the on-state time interval will increase.

# Fig. 1

# Fig. 2

# Fig. 3

# Fig. 4

# Fig. 5

## Fig. 6

## Fig. 7

# Fig. 8

# Fig. 9

# Fig. 10

REGULAR TEMPERATURE CHARACTERISTIC IN LINEAR BURNING MOXA TREATMENT

EP 0 602 267 A1

*Fig. 1 1*

EP 0 602 267 A1

# Fig. 12

| | European Patent Office | EUROPEAN SEARCH REPORT | Application Number |
|---|---|---|---|

EP 92 12 1352

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | FR-A-2 664 497 (PAULE)<br>* page 3, line 2 - line 18; figure 1 * | 1-5 | A61H39/06 |
| A | PATENT ABSTRACTS OF JAPAN<br>vol. 15, no. 256 (C-0845)28 June 1991<br>& JP-A-30 85 167 ( NKK CORP ) 10 April<br>1991<br>* abstract * | 1-10 | |
| A | PATENT ABSTRACTS OF JAPAN<br>vol. 13, no. 431 (C-640)(3779) 26<br>September 1989<br>& JP-A-11 66 757 ( HIROSHI NAKAJIMA ) 30<br>June 1989<br>* abstract * | 1-9 | |

TECHNICAL FIELDS
SEARCHED (Int. Cl.5)

A61B
A61H

The present search report has been drawn up for all claims

| Place of search | Date of completion of the search | Examiner |
|---|---|---|
| BERLIN | 24 AUGUST 1993 | MONNE E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................................
& : member of the same patent family, corresponding document

EPO FORM 1503 03.82 (P0401)